# EUROPÄISCHE PATENTSCHRIFT

(11) **EP 3 073 945 B1**
(45) Veröffentlichungstag und Bekanntmachung des Hinweises auf die Patenterteilung: **02.01.2019**
(21) Anmeldenummer: 14805532.0
(22) Anmeldetag: 24.11.2014
(51) Int. Cl.: A61B 17/221, A61B 17/50, A61B 17/00, A61B 17/22

(54) **SCHLINGENVORRICHTUNG ZUM EINFANGEN EINES OBJEKTES**
SNARE DEVICE FOR CATCHING AN OBJECT
DISPOSITIF FORMANT UNE ANSE POUR CAPTURER UN OBJET

(30) Priorität: 29.11.2013 DE 202013105451 U
(43) Veröffentlichungstag der Anmeldung: 05.10.2016
(73) Patentinhaber: PFM Medical AG, 50996 Köln (DE)
(72) Erfinder: KRAMANN, Bernhard, 66424 Homburg/Saar (DE)
(74) Vertreter: Hohendorf, Carsten
(86) Internationale Anmeldenummer: PCT/EP2014/075388
(87) Internationale Veröffentlichungsnummer: WO 2015/078806

(56) Entgegenhaltungen:
- EP-A1- 1 974 692
- WO-A1-00/16703
- WO-A1-2011/094261
- US-A- 5 342 371
- US-A1- 2013 035 699

## Beschreibung

Die Erfindung betrifft eine Schlingenvorrichtung gemäß Anspruch 1 zum Einfangen eines Objektes.

Schlingenvorrichtungen zum Einfangen von Objekten aus Formgedächtnismaterial, welche in einem hohlen Element gelagert und beweglich gegenüber diesem ausgebildet sind, sind aus dem Stand der Technik bekannt.
In der DE 195 14 534 C2 und der EP 1 113 755 B1 sind Fangschlingen beschrieben, welche mittels eines minimalinvasiven Eingriffs das Extrahieren von Objekten aus dem menschlichen oder tierischen Körper ermöglichen. Diese weisen jeweils eine Schlinge aus Formgedächtnismaterial auf, welche um ein zu extrahierendes Objekt gelegt werden kann. Ist das einzufangende Objekt von der Schlinge umschlossen, wird die Schlinge zugezogen, wodurch das Objekt von der Schlinge festgehalten wird und das Objekt extrahiert werden kann. Mit derartigen Fangschlingen können Objekte, insbesondere Fragmente von Kathetern oder von Führungsdrähten aus vielen unterschiedlichen Richtungen eingefangen werden. Voraussetzung hierfür ist, dass sich das Objekt mit der Schlinge umfassen lässt. Hierzu muss das Objekt ein zugängliches Ende aufweisen, über welches die Schlinge gestülpt werden kann. Objekte, deren Enden nicht zugänglich sind - dies wird im Folgenden nicht-sondierbar genannt - können diese Fangschlingen nicht verwenden.

Sondierbar im Sinne der Erfindung bedeutet folglich, dass ein einzufangendes Objekt mit der Fangschlinge einfangbar ist. Hierzu muss das Objekt ein zugängliches Ende aufweisen, welches mit der Schlinge umfasst bzw. über welches die Schlinge gestülpt werden kann. Die Schlinge wird zugezogen, so dass das Objekt von der Schlinge fest umschlossen ist. Durch ein Zurückziehen der Schlinge bzw. der Schlingenvorrichtung kann das Objekt aus dem menschlichen bzw. tierischen Körper extrahiert werden. Der Begriff "sondierbar" wird im Folgenden in dieser beschriebenen Weise verwendet.

Des Weiteren beschreiben die EP 2 052 688 B2 und die DE 10 2006 053 448 A1 Schlingenvorrichtungen, welche einen Verriegelungsdraht aufweisen, der durch die Schlinge der Schlingenvorrichtung geschoben werden kann, um ein Objekt zwischen einem Teil der Schlinge und dem sich durch die Schlinge erstreckenden Verriegelungsdraht einzufangen. Mit diesen Schlingenvorrichtungen können Objekte eingefangen werden, deren Enden nicht-sondierbar sind. Indem die Schlinge neben das einzufangende Objekt gelegt wird und der Verriegelungsdraht auf der gegenüberliegenden Seite das Objekt umschließt, wobei der Verriegelungsdraht in die Schlinge eingreift, ist das Objekt folglich mittels Schlinge und dem Verriegelungsdraht umschlossen. Das Objekt ist eingefangen und kann extrahiert werden. Nachteilig an derartigen Schlingenvorrichtungen ist, dass die mit den Schlingenvorrichtungen verwendeten hohlen Elemente einen großen Durchmesser aufweisen, da diese zusätzlich zu der Schlinge noch einen Verriegelungsdraht aufweisen.

Die WO 2011/094261 A1 offenbart eine Schlingenvorrichtung gemäß dem Oberbegriff des Anspruchs 1. Bei dem Einfangen eines Objekts wird die Schlinge über ein offenes Ende des einzufangenden Objekts gelegt.

Im klinischen Alltag hat sich gezeigt, dass Situationen, in denen die Enden der zu extrahierenden Objekte nicht-sondierbar sind, relativ häufig anzutreffen sind. Bei etwa jedem zehnten Eingriff liegt dieser Fall vor. Mittels eines gesondert einzuführenden Pigtail-Katheters kann versucht werden, sich mit dem Ende des Pigtail-Katheters an dem Objekt, beispielsweise einem Fragment eines Katheters, einzuhaken und anschließend mit dem Katheter eine Zugbewegung auszuführen, so dass die Möglichkeit besteht, ein Ende des Objektes durch die Verschiebung in einen freiliegenden Bereich zu sondieren. Der freiliegende Bereich kann beispielsweise durch eine benachbarte Vene oder eine Körperöffnung bereit gestellt sein.

Um Objekte, deren Enden nicht-sondierbar sind, einzufangen und zu extrahieren, sind aus dem Stand der Technik speziell auf diesen Anwendungsfall ausgerichtete Zangen, bzw. spezielle Fangschlingen zur Extraktion der Objekte bekannt. Diese speziellen Instrumente sind ausschließlich für den beschriebenen Anwendungsfall für Objekte mit nicht-sondierbaren Enden vorgesehen. Sie weisen spezielle Haken bzw. hakenartige Schlingen auf, mit denen das Objekt angehakt werden kann und anschließend eine Fixierung des Objektes durch einen Verriegelungsdraht erfolgen kann. Der Verriegelungsdraht verschränkt sich zwischen dem Objekt und der hakenartigen Schlinge, so dass das Objekt verriegelt ist und geborgen werden kann.

Nachteilig bei den aus dem Stand der Technik bekannten Lösungen ist, dass diese speziellen Instrumente zum Einfangen von Objekten mit nicht-sondierbaren Enden erst dann eingesetzt werden, wenn mit einer konventionellen Schlingenvorrichtung das Einfangen fehlgeschlagen ist, also ein Objekt mit nicht-sondierbaren Enden vorliegt. Die konventionelle Schlingenvorrichtung muss dann zunächst aus dem menschlichen oder tierischen Körper entfernt werden und das spezielle Instrument gesondert eingeführt werden. Hieraus ergibt sich ein weiterer Nachteil, der darin begründet ist, dass das Kaliber der Spezialinstrumente größer ist als das Kaliber der konventionellen Schlingenvorrichtung. Dies vergrößert ebenfalls die Punktionsstelle, was zu Komplikationen an der Punktionsstelle führen kann. Die Folgen können die Entwicklung von Thrombosen und/oder von schwer zu stillenden Nachblutungen sein.

Ausgehend von diesem Stand der Technik besteht die Aufgabe der Erfindung darin, die Leistungsfähigkeit der aus dem Stand der Technik bekannten Schlingenvorrichtungen zu verbessern, insbesondere um über das Einfangen von Objekten mit sondierbaren Enden hinausgehend das Einfangen von Objekten mit nicht-sondierbaren Enden zu ermöglichen.

Eine weitere Aufgabe der Erfindung besteht darin, eine Schlingenvorrichtung zu schaffen, deren Kaliber auch bei dem Anwendungsfall des Einfangens von Objekten mit nicht-sondierbaren Enden gering ist, insbesondere um Komplikationen, die durch einen größeren Kaliber der Schlingenvorrichtungen hervorgerufen werden können, für den Patienten zu vermeiden.

Zur technischen Lösung dieser Aufgabe wird mit der Erfindung eine Schlingenvorrichtung gemäß Anspruch 1 vorgeschlagen.

Dabei liegt der Erfindung die Erkenntnis zugrunde, dass ein Umgreifen eines Objektes bei dem Einfangen von Objekten mit nicht-sondierbaren Enden nicht mit Hilfe eines zusätzlichen Drahtes, beispielsweise einem Führungs- oder Verriegelungsdrahtes oder ähnlichen Mechanismen durchgeführt werden muss, sondern dass dies mit dem hohlen Element selber realisiert werden kann. Somit kann mit der vorgeschlagenen Lösung sowohl ein Einfangen von Objekten mit sondierbaren Enden, wie auch von Objekten mit nicht-sondierbaren Enden durchgeführt werden. Es kann folglich auf den Einsatz von aus dem Stand der Technik bekannten und auf diesem Zweck gerichteten Spezialinstrumenten verzichtet werden. Der Vorteil, der sich hierdurch ergibt, ist, dass das verwendete hohle Element einen geringeren Kaliber aufweist, da kein zusätzlicher Kanal für den Führungs- bzw. Verriegelungsdraht notwendig ist.
Als Schlinge der Schlingenvorrichtung wird vorgeschlagen, die aus der EP 1 113 755 B1 bekannte Schlinge zu verwenden. Diese Schlinge ist zumindest teilweise doppelt gelegt und bildet zwei Schlingenteile aus, die zueinander so ausgerichtet sind, dass sie eine innere Öffnung und insbesondere diese umgebende Spalten in einer eingeprägten Konfiguration aufweist. Zusätzlich ist die Ebene der Schlinge in der eingeprägten Konfiguration gegenüber dem mit der Schlinge verwendeten hohlen Element in einem Winkel abgewinkelt.

Die Erfindung sieht vor, dass die Schlinge relativ zu dem hohlen Element um 360° drehbar ist. Durch das Drehen der Schlinge kann diese derart manipuliert werden, dass sie entweder aus der endständigen Austrittsöffnung des hohlen Elementes oder aus der seitlichen Austrittsöffnung des hohlen Elementes austritt. In den Fällen, in denen die Enden von einzufangenden Objekten nicht-sondierbar sind, wird seitens eines Operateurs die Schlinge aus der seitlichen Austrittsöffnung des hohlen Elementes herausgeschoben und das hohle Element in eine derartige Position bewegt, dass sich das einzufangende Objekt zwischen dem hohlen Element und der ausgetretenen Schlinge befindet. Um das Objekt einzufangen, wird die Schlinge derart zurück in das hohle Element gezogen, dass sich die Schlinge über das hohle Element stülpt, so dass das einzufangende Objekt umfasst und verriegelt ist.

Die Schlinge ist mittels einer Manipulationsvorrichtung drehbar, insbesondere mittels eines Torquers, welcher an dem proximalen Ende der Schlinge angeordnet ist. Darüber hinaus ist die Schlinge zusätzlich mittels der Manipulationsvorrichtung in das hohle Element hineinziehbar und aus diesem herausschiebbar. Die Verwendung einer derartigen Manipulationsvorrichtung erleichtert die Handhabung der Schlinge, da diese zumeist aus einem sehr dünnen Material besteht. In der Manipulationsvorrichtung selber wird die Schlinge über ein Klemmmittel arretiert. Dieses kann gelöst werden, falls die Manipulationsvorrichtung nicht mehr benötigt wird.
Eine weitere Ausgestaltung der Erfindung sieht vor, dass die seitliche Austrittsöffnung des hohlen Elementes um etwa 1 cm bis etwa 3 cm gegenüber dem distalen Ende des hohlen Elementes zurückversetzt ist, bevorzugt um etwa 2 cm. Dies gewährleistet einen hinreichenden Abstand zwischen der seitlichen Austrittsöffnung und dem distalen Ende des hohlen Elementes, so dass mittels der erfindungsgemäßen Vorrichtung das hohle Element zur Verriegelung bzw. zum Umfassen von Objekten mit nicht-sondierbaren Enden verwendet werden kann. Eine bevorzugte Ausgestaltung der Erfindung sieht vor, dass die seitliche Austrittsöffnung des hohlen Elementes in Form eines länglichen Schlitzes ausgebildet ist. Eine derartige Formgebung der seitlichen Austrittsöffnung erleichtert das Austreten der Schlinge aus der seitlichen Austrittsöffnung des hohlen Elementes.
In einer weiteren Ausgestaltung besteht das Formgedächtnismaterial der Schlinge aus Metall, insbesondere aus Nitinol oder aus Kunststoff.
Vorteilhafterweise weist das hohle Element einen Durchmesser von etwa zwei French bis etwa acht French auf, bevorzugt von etwa vier French bis etwa sechs French. Der Durchmesser des mit der Schlingenvorrichtung verwendeten hohlen Elementes ist folglich signifikant geringer, als der Durchmesser der aus dem Stand der Technik bekannten Spezialinstrumente zum Einfangen von Objekten bzw. Fremdkörpern mit nicht-sondierbaren Enden. Ein wesentlicher Vorteil durch den geringeren Durchmesser des hohlen Elementes ist, dass Komplikationen, die mit hohlen Elementen größerer Durchmesser auftreten können, an der Punktionsstelle vermieden werden. Insbesondere die Risiken von auftretenden Thrombosen und/oder schwer zu stillenden Nachblutungen an der Punktionsstelle werden vermindert.
Die erfindungsgemäße Schlingenvorrichtung weist an der Schlinge eine Markierung auf, um die Schlinge unter Darstellungsvorrichtungen sichtbar zu machen. Während der Durchführung eines Eingriffs, daher um ein Objekt bzw. einen Fremdkörper zu bergen bzw. einzufangen und zu extrahieren, ist es vorteilhaft, wenn die Schlinge eine Markierung aufweist, die unter einer Darstellungsvorrichtung die Schlinge sichtbar macht. Möglich ist dies beispielsweise durch die Verwendung eines Fluoroskops, eines MRTs bzw. durch weitere elektronische Darstellungsvorrichtungen, die Markierungen beispielsweise mittels einen Röntgengerätes oder einer Sonographie an der Schlinge sichtbar machen. Die Markierungen selber können beispielsweise Magnetresonanzmarkierungen sein.
Weitere Einzelheiten, Merkmale und Vorteile der Erfindung werden nachfolgend anhand des in den Figuren dargestellten Ausführungsbeispiels näher erläutert. Dabei zeigen
- Fig. 1: eine Ausgestaltung eines erfindungsgemäßen Katheters;
- Fig. 2: eine Ausgestaltung einer Schlingenvorrichtung mit vollständig ausgetretener Schlinge aus der endständigen Austrittsöffnung des Katheters;
- Fig. 3: die erfindungsgemäße Schlingenvorrichtung mit teilweise ausgetretener Schlinge aus der seitlichen Austrittsöffnung des Katheters;
- Fig. 4: die Ausgestaltung der erfindungsgemäßen Schlingenvorrichtung mit vollständig ausgetretener Schlinge aus der seitlichen Austrittsöffnung des Katheters;
- Fig. 5: die Schlingenvorrichtung gemäß Fig. 4, wobei die Schlinge um 180° gegenüber dem Katheter gedreht ist;
- Fig. 6: die Schlingenvorrichtung gemäß Fig. 5 mit teilweise in den Katheter hineingezogener Schlinge und
- Fig. 7a und b: die erfindungsgemäße Schlingenvorrichtung mit seitlich eingefangenem Fremdkörper.

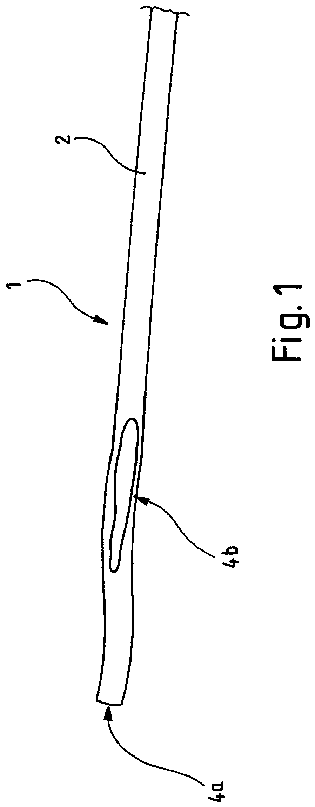

In Fig. 1 ist ein Katheter (2) einer Ausgestaltung einer erfindungsgemäßen Schlingenvorrichtung (1) in einer Seitenansicht dargestellt. Der Katheter (2) weist neben der endständigen Austrittsöffnung (4a) eine seitliche Austrittsöffnung (4b) auf. Die seitliche Austrittsöffnung (4b) ist in Form eines länglichen Schlitzes ausgebildet. Die Schlinge (3) befindet sich vollständig in dem Katheter (2).

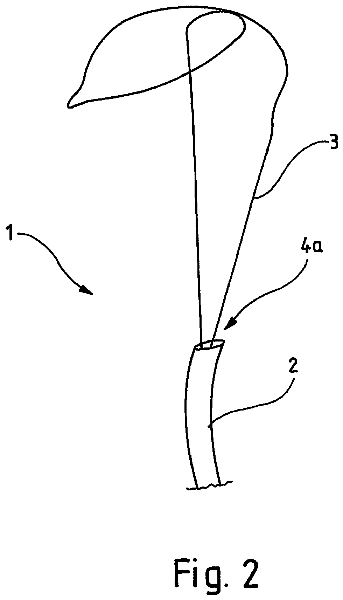

In Fig. 2 ist die Schlingenvorrichtung (1) dargestellt, wobei die Schlinge (3) der Schlingenvorrichtung (1) vollständig aus der endständigen Austrittsöffnung (4a) aus dem Katheter (2) ausgetreten ist. Dies stellt den konventionellen Austritt dar, der bereits aus dem Stand der Technik der EP 1 113 755 B1 bekannt ist.

Bei dem Austreten der Schlinge (3) aus der endständigen Austrittsöffnung (4a) des dem Katheters (2) können einzufangende Fremdkörper (5), wie beispielsweise Katheterfragmente, Drähte oder dergleichen ausschließlich eingefangen werden, wenn diese ein zugängliches Ende aufweisen, welches mittels der Schlinge (3) sondierbar ist. Hierzu wird die Schlinge (3) über den einzufangenden Fremdkörper (5) gestülpt und zurück in den Katheter (2) gebracht, wodurch sich die Schlinge (3) zuzieht und den Fremdkörper (5) umfasst. Der Fremdkörper (5) kann anschließend extrahiert werden.

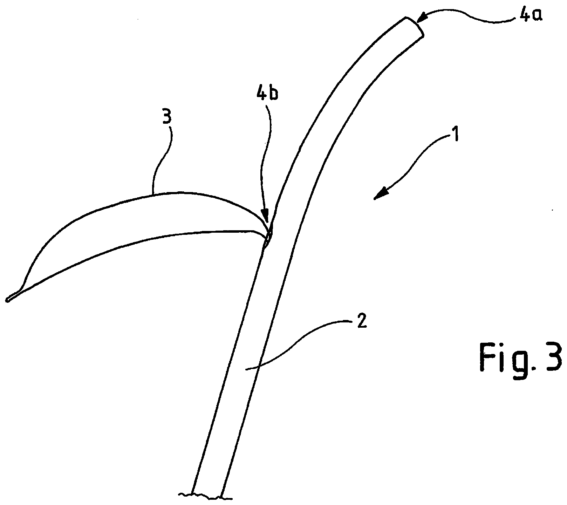

Falls der Fremdkörper (5) auf diese Weise nicht einfangbar ist und demzufolge kein frei zugängliches Ende aufweist, wird die Schlinge (3) in den Katheter (2) zurückgezogen und mittels eines Torquers, welcher am proximalen Ende der Schlinge angeordnet ist und einem Untersucher bzw. einem Operateur ermöglicht, die Schlinge relativ zu dem Katheter (2) zu drehen und in den Katheter (2) hinein bzw. aus dem Katheter (2) herauszuschieben, durch Drehen derart manipuliert, dass die Schlinge (3) nun aus der seitlichen Austrittsöffnung (4b) des Katheters austritt. Dies ist in Fig. 3 dargestellt.

Die Schlinge (3) ist in ihrer eingeprägten Ursprungsform (vgl. Fig. 2) derart geformt, dass das längliche proximale Teilstück der Schlinge (3) in einem vorbestimmten Winkel abgewinkelt ist. Durch diese Abwinkelung und dem Bestreben des Formgedächtnismaterials der Schlinge (3) in die eingeprägte Ursprungsform zurückzukehren, kann die Schlinge (3) also derart manipuliert werden, dass ein Austritt der Schlinge (3) aus der seitlichen Austrittsöffnung (4b) möglich ist, oder falls dies nicht gewünscht ist, durch Drehen am Torquer derart manipuliert werden, dass diese nicht aus der seitlichen Austrittsöffnung (4b) des Katheters (2) austritt, sondern aus der endständigen Austrittsöffnung (4a) des Katheters (2) Austritt.

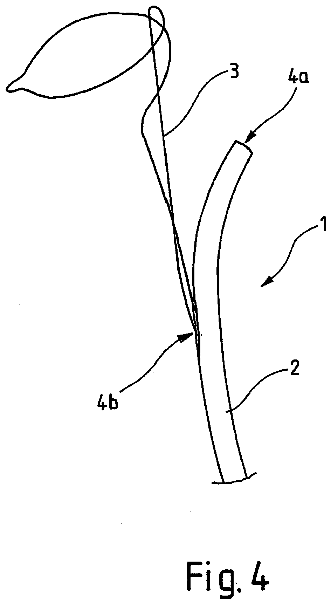

Wie in Fig. 3 dargestellt ist, ist die Schlinge (3) teilweise aus der seitlichen Austrittsöffnung (4b) ausgetreten. Wird die Schlinge (3) durch den Torquer derart bewegt, dass die Schlinge (3) weiter aus dem Katheter (2) austritt, nimmt die Schlinge (3) ihre eingeprägte Ursprungsform ein, was in Fig. 4 dargestellt ist. Die Schlinge (3) ist nun vollständig aus der seitlichen Austrittsöffnung (4b) des Katheters (2) ausgetreten.

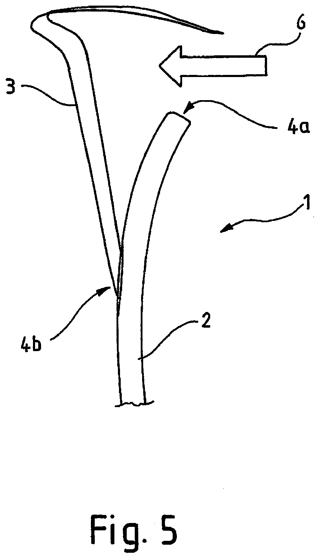

Um ein Einfangen von Objekten, deren Enden nicht-sondierbar sind, zu ermöglichen, muss die Schlinge (3) durch Manipulation am Torquer um 180° gegenüber dem Katheter (2) gedreht werden. Dies ist in Fig. 5 dargestellt.

Um das Einfangen zu realisieren, wird der einzufangende Fremdkörper (5) zwischen dem distalen Ende des Katheters (2) und dem abgewinkelten proximalen Teilstück der Schlinge (3) platziert. Dies ist durch den Pfeil (6) dargestellt.

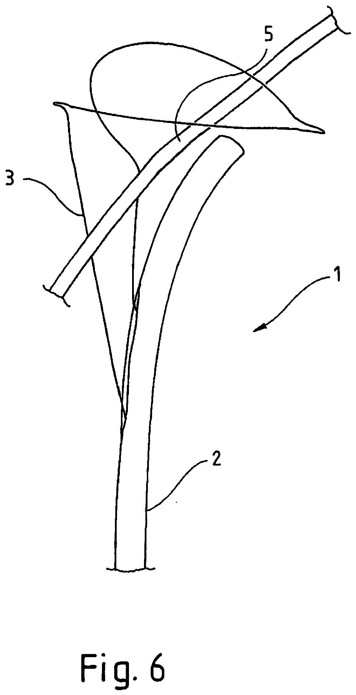

Ist der einzufangende Fremdkörper (5) zwischen dem distalen Ende des Katheters (2) und dem abgewinkelten Teilstück der Schlinge (3) platziert, wird die Schlinge (3) durch Manipulation am Torquer langsam in den Katheter (2) zurückgezogen, wobei sich das abgewinkelte proximale Teilstück der Schlinge über das distale Ende des Katheters (2) stülpt. Fig. 6 zeigt diesen Schritt des Einfangens.

Der einzufangende Fremdkörper (5), dessen Enden nicht sondierbar sind, befindet sich zwischen dem distalen Ende des Katheters (2) und dem abgewinkelten proximalen Teilstück der Schlinge (3). Das abgewinkelte proximale Teilstück der Schlinge (3) ist derart gegenüber dem Katheter (2) ausgerichtet, dass es sich bei dem Zurückziehen der Schlinge (3) durch Manipulation am Torquer in den Katheter (2) zurück über das distale Ende des Katheters (2) stülpt. Der einzufangende Fremdkörper (5) ist somit von der Schlinge (3) und dem Katheter (2) umschlossen.

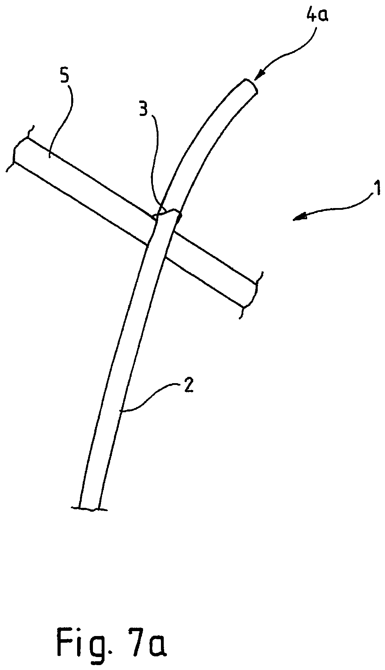

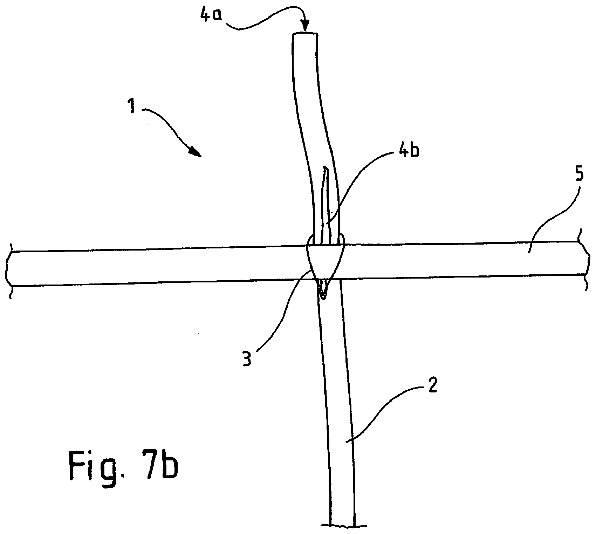

In Fig. 7a ist dargestellt, dass der Fremdkörper (5), vorliegend ein Katheterstück, vollständig von der Schlinge (3) und dem Katheter (2) umfasst ist, so dass dieser extrahiert werden kann, was über ein Zurückziehen der gesamten Schlingenvorrichtung (1) erreicht wird. Gegenüber der Darstellung in Fig. 7a ist in Fig. 7b der eingefangene Fremdkörper (5) in einer Ansicht von vorne dargestellt.

Die seitliche Austrittsöffnung (4b) des Katheters (2) ist zu erkennen. Die Schlinge (3) ist derart in den Katheter (2) zurückgezogen, dass der eingefangene Fremdkörper (5) vollständig von der Schlinge (3) und dem Katheter (2) umfasst ist und gehalten wird.

Das in den Figuren der Zeichnung dargestellte und beschriebene Ausführungsbeispiel dient lediglich der Erläuterung der Erfindung und ist für diese nicht beschränkend.

### Bezugzeichenliste

- 1: Schlingenvorrichtung
- 2: Katheter
- 3: Schlinge
- 4a: Austrittsöffnung (endständig/distal)
- 4b: Austrittsöffnung (seitlich)
- 5: Fremdkörper
- 6: Fremdkörper-Platzierung

## Patentansprüche

1. Schlingenvorrichtung (1) zum Einfangen eines Objektes, insbesondere zum Einfangen von Objekten oder Fremdkörpern (5) im menschlichen oder tierischen Körpern, umfassend ein hohles Element, insbesondere einen Katheter (2), und eine Schlinge (3) aus einem flexiblen elastischen Formgedächtnismaterial, wobei die Schlinge (3) relativ zu dem hohlen Element beweglich und in das hohle Element hineinziehbar und aus diesem herausschiebbar ist, und wobei die Schlinge (3) eine eingeprägte Ursprungsform aufweist, in welche die Schlinge (3) bei dem Herausschieben aus dem hohlen Element durch die bei dem Hineinziehen herbeigeführten Materialspannungen zurückkehrt,
wobei das hohle Element eine Austrittsöffnung (4b) aufweist, welche seitlich an dem hohlen Element angeordnet und gegenüber dem distalen Ende des hohlen Elementes zurückversetzt ist und durch welche die Schlinge (3) aus dem hohlen Element herausschiebar und in das hohle Element hineinziehbar ist,
**dadurch gekennzeichnet, dass** die Schlinge (3) mittels einer Manipulationsvorrichtung relativ zu dem hohlen Element um 360° drehbar ist und wobei die Schlinge (3) in der eingeprägten Ursprungsform derart geformt ist, dass das längliche proximale Teilstück der Schlinge (3) in einem vorbestimmten Winkel abgewinkelt ist,
so dass sich das einzufangende Objekt zwischen dem hohlen Element und dem abgewinkelten proximalen Teilstück der ausgetretenen Schlinge (3) befindet und sich das abgewinkelte proximale Teilstück der Schlinge (3) beim Zurückziehen über das hohle Element stülpt.

2. Schlingenvorrichtung (1) nach Anspruch 1, **dadurch gekennzeichnet, dass** die Manipulationsvorrichtung ein Torquer ist, welcher in dem Bereich des proximalen Endes der Schlinge (3) angeordnet ist.

3. Schlingenvorrichtung (1) nach Anspruch 1 oder Anspruch 2, **dadurch gekennzeichnet, dass** die Schlinge (3) mittels der Manipulationsvorrichtung in das hohle Element hineinziehbar und aus dem hohlen Element herausschiebbar ist.

4. Schlingenvorrichtung (1) nach einem der Ansprüche 1 bis 3, **dadurch gekennzeichnet, dass** die seitliche Austrittsöffnung (4b) des hohlen Elementes um etwa 1 cm bis etwa 3 cm gegenüber dem distalen Ende des hohlen Elementes zurückversetzt ist, bevorzugt um etwa 2 cm.

5. Schlingenvorrichtung (1) nach einem der Ansprüche 1 bis 4, **dadurch gekennzeichnet, dass** die seitliche Austrittsöffnung (4b) des hohlen Elementes in Form eines länglichen Schlitzes ausgebildet ist.

6. Schlingenvorrichtung (1) nach einem der Ansprüche 1 bis 5, **dadurch gekennzeichnet, dass** das Formgedächtnismaterial der Schlinge (3) aus Metall, insbesondere aus Nitinol, oder aus Kunststoff besteht.

7. Schlingenvorrichtung (1) nach einem der Ansprüche 1 bis 6, **dadurch gekennzeichnet, dass** das hohle Element einen Durchmesser von etwa 2 French bis etwa 8 French aufweist, bevorzugt von etwa 4 French bis etwa 6 French.

8. Schlingenvorrichtung (1) nach einem der Ansprüche 1 bis 7, **dadurch gekennzeichnet, dass** an der Schlinge (3) eine Markierung vorgesehen ist, um die Schlinge unter Darstellungsvorrichtungen sichtbar zu machen.

## Claims

1. Snare device (1) for catching an object, in particular for catching objects or foreign bodies (5) in a human or animal body, comprising a hollow element, in particular a catheter (2), and a snare (3) of a flexible elastic shape-memory material, wherein the snare (3) can be moved relative to the hollow element and can be pulled into the hollow element and can be pushed out of the hollow element, and wherein the snare (3) has a memorized original shape, to which the snare (3) returns, when pushed out of the hollow element, by means of the material stresses caused when being pulled in,
wherein the hollow element has an outlet opening (4b), which is arranged laterally on the hollow element and which is set back from the distal end of the hollow element and through which the snare (3) can be pushed out of the hollow element and can be pulled into the hollow element,
**characterized in that** the snare (3) is rotatable by a manipulation device relative to the hollow element by 360° and wherein the snare (3) in the memorized original shape is formed such that the elongated proximal portion of the snare (3) is angled in a predetermined angle, so that the object to be caught is positioned between the hollow element and the angled proximal portion of the pushed out snare (3) and the angled proximal portion of the snare (3) is put over the hollow element when being pulled in.

2. Snare device (1) according to claim 1, **characterized in that** the manipulation device is a torquer, which is arranged at the proximal end of the snare (3).

3. Snare device (1) according to claim 1 or claim 2, **characterized in that** the snare (3) can be pushed out of the hollow element and can be pulled into the hollow element by the manipulation device.

4. Snare device (1) according to one of claims 1 to 3, **characterized in that** the outlet opening (4b) of the hollow element is set back from the distal end of the hollow element by about 1 cm to about 3 cm, preferably by about 2 cm.

5. Snare device (1) according to one of claims 1 to 4, **characterized in that** the lateral outlet opening (4b) of the hollow element is formed in the shape of an elongated slot.

6. Snare device (1) according to one of claims 1 to 5, **characterized in that** the shape-memory material of the snare (3) consist of metal, in particular nitinol, or of plastics.

7. Snare device (1) according to one of claims 1 to 6, **characterized in that** the hollow element has a diameter of about 2 French to about 8 French, preferably of about 4 French to about 6 French.

8. Snare device (1) according to one of claims 1 to 7, **characterized in that** a marker is arranged on the snare (3), to make the snare visible under displaying devices.

## Revendications

1. Dispositif d'anse (1) permettant de saisir un objet, en particulier de saisir des objets ou des corps étrangers (5) dans un corps humain ou animal, comprenant un élément creux, en particulier un cathéter (2), et une anse (3) d'un matériau souple à mémoire de forme élastique, dans lequel l'anse (3) peut être déplacée par rapport à l'élément creux et peut être tirée dans l'élément creux et peut être poussée hors de l'élément creux, et dans lequel l'anse (3) a une forme d'origine mémorisée, à laquelle l'anse (3) revient, lorsqu'elle est poussée hors de l'élément creux, au moyen des contraintes de matériau provoquées lorsqu'elle est tirée à l'intérieur,
dans lequel l'élément creux présente une ouverture de sortie (4b), qui est disposée de manière latérale sur l'élément creux et qui est en retrait par rapport à l'extrémité distale de l'élément creux et à travers laquelle l'anse (3) peut être poussée hors de l'élément creux et peut être tirée dans l'élément creux,
**caractérisé en ce que** l'anse (3) peut tourner au moyen d'un dispositif de manipulation par rapport à l'élément creux de 360°, et dans lequel l'anse (3), dans la forme d'origine mémorisée, est formée telle que la partie proximale allongée de l'anse (3) est inclinée selon un angle prédéterminé, de sorte que l'objet à saisir est positionné entre l'élément creux et la partie proximale inclinée de l'anse (3) poussée vers l'extérieur et la partie proximale inclinée de l'anse (3) est placée au-dessus de l'élément creux lorsqu'elle est tirée vers l'intérieur.

2. Dispositif d'anse (1) selon la revendication 1, **caractérisé en ce que** le dispositif de manipulation est un dispositif de couple, qui est disposé au niveau de l'extrémité proximale de l'anse (3).

3. Dispositif d'anse (1) selon la revendication 1 ou la revendication 2, **caractérisé en ce que** l'anse (3) peut être poussée hors de l'élément creux et peut être tirée dans l'élément creux par le dispositif de manipulation.

4. Dispositif d'anse (1) selon l'une des revendications 1 à 3, **caractérisé en ce que** l'ouverture de sortie (4b) de l'élément creux est en retrait de l'extrémité distale de l'élément creux d'environ 1 cm à environ 3 cm, de préférence d'environ 2 cm.

5. Dispositif d'anse (1) selon l'une des revendications 1 à 4, **caractérisé en ce que** l'ouverture de sortie latérale (4b) de l'élément creux est formée sous la forme d'une fente allongée.

6. Dispositif d'anse (1) selon l'une des revendications 1 à 5, **caractérisé en ce que** le matériau à mémoire de forme de l'anse (3) se compose de métal, en particulier de nitinol, ou de plastique.

7. Dispositif d'anse (1) selon l'une des revendications 1 à 6, **caractérisé en ce que** l'élément creux a un diamètre d'environ 2 French à environ 8 French, de préférence d'environ 4 French à environ 6 French.

8. Dispositif d'anse (1) selon l'une des revendications 1 à 7, **caractérisé en ce qu'**un marqueur est disposé sur l'anse (3), afin de rendre l'anse visible sous des dispositifs d'affichage.
